# EUROPEAN PATENT APPLICATION

(11) **EP 4 349 352 A1**
(43) Date of publication of application: **10.04.2024**
(21) Application number: 22816405.9
(22) Date of filing: 27.05.2022
(51) Int. Cl.: A61K 35/747, A61K 9/00, A61P 37/00, A61P 3/00, A23L 33/135, A61K 8/99, A61Q 19/00

(54) **LACTOBACILLUS ACIDOPHILUS-DERIVED VESICLE AND USE THEREOF**

(30) Priority: 03.06.2021 KR 20210072078; 30.11.2021 KR 20210168794
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2022/007609
(87) International publication number: WO 2022/255746

(57) **Abstract**

The present invention relates to *Lactobacillus acidophilus*-derived vesicles and a use thereof, and more specifically, to a composition for alleviating, preventing, or treating an inflammatory disease and cancer, which includes *Lactobacillus acidophilus*-derived vesicles as an active ingredient.

## Description

### [Technical Field]

The present invention relates to *Lactobacillus acidophilus-derived* vesicles and a use thereof, and more particularly, to a composition for preventing or treating an inflammatory disease, which comprises *Lactobacillus acidophilus-derived* vesicles as an active ingredient.

This application claims priority to and the benefit of Korean Patent Application Nos. 10-2021-0072078 and 10-2021-0168794 filed in the Korean Intellectual Property Office on June 3, 2021 and November 30, 2021, respectively, and all the contents disclosed in the specification and drawings of the applications are incorporated in this application.

### [Background Art]

As we enter the 21st century, the significance of acute infectious diseases, previously recognized as contagious diseases, has diminished, whereas the disease pattern has changed such that chronic diseases, have become the major diseases that determine the quality of life and human lifespan. The chronic diseases are characterized by chronic inflammation accompanied by immune dysfunction, and various chronic inflammatory diseases and cancers caused thereby are becoming a major public health problem.

Inflammation is a local or systemic protective mechanism against the damage or infection of cells and tissues, and is typically caused by serial biological responses occurring as humoral mediators that constitute the immune system directly response to the damage or infection or stimulate the local or systemic effector system. As a result of inflammation, cell death occurs, resulting in disease. Examples of a main inflammatory disease include digestive diseases such as gastritis and inflammatory enteritis, oral diseases such as periodontitis, respiratory diseases such as asthma, chronic obstructive pulmonary disease (COPD), and rhinitis, dermatological diseases such as atopic dermatitis, alopecia, acne and psoriasis, musculoskeletal inflammatory disease such as degenerative arthritis, rheumatoid arthritis, gout, and osteoporosis, and cancers, and the like.

Common compositions used to treat or prevent chronic inflammation diseases are largely classified into steroidal and non-steroidal compositions, most of which are often accompanied by various side effects in many cases. Therefore, there is a trend in which interests in a TNF-α and IL-6 inhibitor, which is a pro-inflammatory cytokine known to be a main cause of chronic inflammation diseases, have been recently increased.

However, the development of drugs that have low side effects and may be substantially used for the prevention and treatment of chronic inflammation diseases by effectively suppressing the expression of TNF-α and IL-6 still has been inadequate to date.

### [Disclosure]

### [Technical Problem]

As a result of intensively conducting research to solve the above-mentioned conventional problems, the present inventors confirmed that *Lactobacillus acidophilus-derived* vesicles have an excellent TNF-α and IL-6 inhibiting effect, and thus completed the present invention.

Therefore, the present invention is directed to providing a composition for alleviating, preventing, or treating an inflammatory disease, which comprises *Lactobacillus acidophilus-derived* vesicles as an active ingredient.

However, a technical problem to be achieved by the present invention is not limited to the aforementioned problems, and the other problems that are not mentioned may be clearly understood by a person skilled in the art from the following description.

### [Technical Solution]

To achieve the purpose of the present invention, when the present inventors confirmed that *Lactobacillus acidophilus* was cultured, and vesicles were isolated and then administrated orally to a mouse, it was confirmed that the vesicles were distributed to organs such as the stomach, the small intestine, the large intestine, the lung, the liver, and the central nervous system. In addition, when a mouse macrophage cell line was treated with the vesicles, it was confirmed that an inflammation-inducing effect is not exhibited, and the expression of TNF-α and IL-6, which was increased by an inflammatory factor, that is, E. coli-derived vesicles, is significantly suppressed.

Thus, the present invention is to provide a pharmaceutical composition for preventing or treating an inflammatory disease, comprising *Lactobacillus acidophilus-*derived vesicles as an active ingredient.

In addition, the present invention is to provide a food composition for preventing or alleviating an inflammatory disease, comprising *Lactobacillus acidophilus-derived* vesicles as an active ingredient.

In addition, the present invention is to provide an inhalant composition for preventing or treating an inflammatory disease or a cancer, comprising *Lactobacillus acidophilus-derived* vesicles as an active ingredient.

In addition, the present invention is to provide a cosmetic composition for preventing or alleviating an inflammatory skin disease, comprising *Lactobacillus acidophilus-derived* vesicles as an active ingredient.

In one embodiment of the present invention, the inflammatory disease may comprise the following diseases, but the present invention is not limited thereto:
one or more inflammatory skin diseases selected from the group consisting of eczema, atopic dermatitis, psoriasis, acne, alopecia, contact dermatitis, seborrheic dermatitis, and mastocytosis;
one or more inflammatory oral diseases selected from the group consisting of caries, gingivitis, and periodontitis;
one or more inflammatory gastrointestinal diseases selected from the group consisting of ulcerative colitis, Crohn's disease, intestinal Behcet's disease, indeterminate colitis, chronic gastritis, chronic peptic ulcers, celiac disease, and inflammatory bowel disease;
one or more inflammatory liver-biliary-pancreatic diseases selected from the group consisting of non-alcoholic steatohepatitis (NASH), alcoholic steatohepatitis, liver cirrhosis, cholangitis, cholecystitis, and pancreatitis;
one or more inflammatory nasal diseases selected from the group consisting of rhinitis, paranasal sinusitis, and sinusitis;
one or more inflammatory lung diseases selected from the group consisting of bronchitis, asthma, chronic obstructive pulmonary disease (COPD), emphysema, cystic fibrosis, pneumonia, chronic interstitial pneumonitis, bronchopulmonary dysplasia, and idiopathic pulmonary fibrosis;
one or more inflammatory cardiovascular diseases selected from the group consisting of hyperinsulinemia, dyslipidemia, arrhythmia, atherosclerosis, angina, metabolic syndrome, myocardial infarction, cardiomyopathy, stroke, ischemic heart disease, heart failure, thrombosis, and atherosclerosis;
one or more inflammatory musculoskeletal diseases selected from the group consisting of atony, muscular atrophy, muscular dystrophy, myasthenia gravis, cachexia, gout, sarcopenia, osteoporosis, Paget's disease, rheumatoid arthritis, and osteoarthritis;
one or more inflammatory central nervous system diseases selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), anxiety disorder, post-traumatic stress disorder (PTSD), panic disorder, depression, Autism spectrum disorders, attention deficit/hyperactivity disorder (ADHD), schizophrenia, dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy; and
one or more chronic inflammatory eye diseases selected from the group consisting of proliferative vitreoretinopathy, macular degeneration, pigmentary retinopathy, diabetic retinopathy, choroidal neovascularization, neovascular glaucoma, ischemic optic nerve disorders, retinopathy of prematurity, epidemic keratoconjunctivitis, neovascular iris disease, retrolental fibroplasia, atopic keratitis, superior corneal limbal keratitis, psoriasis keratitis, phlyctenular keratoconjunctivitis, scleritis, and diabetic macular edema.

In another embodiment of the present invention, the inflammatory disease may be a disease mediated by TNF-α or IL-6, but the present invention is not limited thereto.

As another exemplary embodiment of the present invention, the vesicles may be naturally secreted or artificially produced from *Lactobacillus acidophilus,* but the present invention is not limited thereto.

As yet another exemplary embodiment of the present invention, the vesicles may have an average diameter of 10 to 300 nm, but are not limited thereto.

In yet another embodiment of the present invention, the vesicles may be isolated from the culture medium of *Lactobacillus acidophilus* or food cultured by adding *Lactobacillus acidophilus,* but the present invention is not limited thereto.

In addition, the present invention provides a composition for delivering a disease-treating drug, which comprises *Lactobacillus acidophilus-derived* vesicles as an active ingredient.

In one embodiment of the present invention, the disease may be one or more selected from the group consisting of a stomach disease, an intestinal disease, a lung disease, a liver disease, and a central nervous system disease, but the present invention is not limited thereto.

In another embodiment of the present invention, the central nervous system disease may be one or more selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), anxiety disorder, post-traumatic stress disorder (PTSD), panic disorder, depression, Autism spectrum disorders, attention deficit/hyperactivity disorder (ADHD), schizophrenia, dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy, but the present invention is not limited thereto.

In addition, the present invention provides a method for preventing or treating an inflammatory disease, the method comprising administering a composition comprising *Lactobacillus acidophilus-derived* vesicles as an active ingredient to a subject in need thereof.

In addition, the present invention provides a use of a composition comprising *Lactobacillus acidophilus-derived* vesicles as an active ingredient for preventing or treating an inflammatory disease.

In addition, the present invention provides a use of *Lactobacillus acidophilus-*derived vesicles for producing a drug for treating an inflammatory disease.

In addition, the present invention provides a method for preventing, alleviating or treating an inflammatory skin disease, the method comprising administering a composition comprising *Lactobacillus acidophilus-derived* vesicles as an active ingredient to a subject in need thereof.

In addition, the present invention provides a use of a composition comprising *Lactobacillus acidophilus-*derived vesicles as an active ingredient for preventing, alleviating or treating an inflammatory skin disease.

In addition, the present invention provides a use of *Lactobacillus acidophilus-*derived vesicles for preparing a drug for treating an inflammatory skin disease.

In addition, the present invention provides a method of delivering a drug for treating a disease, which comprises administering a composition comprising *Lactobacillus acidophilus-derived* vesicles as an active ingredient to a subject in need thereof.

In addition, the present invention provides a use of a composition comprising *Lactobacillus acidophilus-derived* vesicles carrying a drug for treating a disease as an active ingredient for delivering a disease-treating drug.

In addition, the present invention provides a drug delivery system, which comprises *Lactobacillus acidophilus-derived* vesicles.

In addition, the present invention provides a use of *Lactobacillus acidophilus-*derived vesicles for preparing an agent for drug delivery in cells.

### [Advantageous Effects]

When *Lactobacillus acidophilus-derived* vesicles according to the present invention are orally administrated, they are absorbed through the stomach, the small intestine, and the large intestine, move into the blood through lymphatic vessels, and then are distributed to organs such as the lungs, the liver, and the brain. In addition, since the vesicles can effectively suppress the expression of TNF-α and IL-6, which are mediators causing an inflammatory disease by a pathogenic factor, they are expected to be widely used as therapeutic agents for alleviating, preventing, or treating various inflammatory diseases.

### [Description of Drawings]

FIG. 1 is a view of analyzing the absorption, distribution, and excretion patterns of vesicles over time after orally administering *Lactobacillus acidophilus-*derived vesicles according to one embodiment of the present invention.
FIG. 2 is a view of analyzing the absorption, distribution, and excretion patterns in the lungs, small intestine, and large intestine over time after orally administering *Lactobacillus acidophilus-derived* vesicles according to one embodiment of the present invention.
FIG. 3 is a view of analyzing the absorption, distribution, and excretion patterns in the lungs and liver over time after orally administering *Lactobacillus acidophilus-derived* vesicles according to one embodiment of the present invention.
FIG. 4 is a view of analyzing the absorption, distribution, and excretion patterns in the central nervous system (particularly, the brain) over time after orally administering *Lactobacillus acidophilus-derived* vesicles according to one embodiment of the present invention.
FIG. 5 is a view of analyzing the absorption, distribution, and excretion patterns in the heart and the kidneys over time after orally administering *Lactobacillus acidophilus-derived* vesicles according to one embodiment of the present invention.
FIGS. 6A and 6B are view showing the results of comparing the secretion effects of *E*. *coli*-derived vesicles (*E. coli* EV) and *Lactobacillus acidophilus-derived* vesicles on the secretion of TNF-α and IL-6, which are inflammatory mediators in inflammatory cells, according to one embodiment of the present invention.
FIGS. 7A and 7B are views showing the treatment effects of *Lactobacillus acidophilus-derived* vesicles on TNF-α and IL-6 secretion by *E*. *coli*-derived vesicles (*E. coli* EV), which is a pathogenic factor, in inflammatory cells according to one embodiment of the present invention.

### [Best Mode]

In the present invention, it was confirmed that, when *Lactobacillus acidophilus-derived* vesicles were administrated orally, they were absorbed through the stomach, small intestine, and large intestine, absorbed into blood vessels through lymphatic vessels, and then distributed into organs such as the lungs, liver, and brain. In addition, it was confirmed that the *Lactobacillus acidophilus*-derived vesicles effectively inhibited the secretion of an inflammatory mediator causing an inflammatory disease by a pathogenic factor, thereby exhibiting a significant treatment effect on inflammatory diseases.

Thus, the present invention relates to a composition for preventing, alleviating or treating an inflammatory disease, comprising *Lactobacillus acidophilus-derived* vesicles as an active ingredient.

Hereinafter, the present invention will be described in detail.

In the present invention, *Lactobacillus acidophilus* is one species of the Lactobacillus that is a type of probiotics, and has a rod shape, is resistant to acid, and is known to have intestinal and anti-cancer effects, lower blood cholesterol, and synthesize vitamin B-complex.

As used herein, the "extracellular vesicle" or "vesicle" refers to a structure formed of a nano-sized membrane secreted from various bacteria, and includes, for example, a vesicle derived from gram-negative bacteria such as *E. coli,* which has, an endotoxin (lipopolysaccharide), a toxic protein, and both bacterial DNA and RNA, or a vesicle derived from gram-positive bacteria such as bacteria of the genus *Micrococcus,* which have outer membrane vesicles (OMVs), a protein and a nucleic acid as well as components of a bacterial cell wall, such as peptidoglycan and lipoteichoic acid.

In the present invention, the vesicles encompasses all structures which are naturally secreted from *Lactobacillus acidophilus,* or formed of an artificially produced membrane. The vesicles may be isolated from a culture solution including *Lactobacillus acidophilus* using one or more methods selected from the group consisting of centrifugation, ultracentrifugation, autoclaving, extrusion, sonication, cell lysis, homogenization, freezing-thawing, electroporation, mechanical degradation, chemical treatment, filtration with a filter, gel filtration chromatography, pre-flow electrophoresis, and capillary electrophoresis of the cell culture. In addition, for isolation, washing for removing impurities, and concentration of the obtained vesicles may be further performed.

A method of isolating vesicles from the *Lactobacillus acidophilus* culture medium or fermented food according to the present invention is not particularly limited as long as the culture or fermented food contains vesicles. For example, vesicles may be isolated using a method such as centrifugation, ultracentrifugation, filtration by a filter, gel filtration chromatography, free-flow electrophoresis, or capillary electrophoresis, or a combination thereof, and may further include washing for removing impurities, and concentration of the acquired vesicles.

The vesicles of the present invention may be isolated from a *Lactobacillus acidophilus* culture medium or food manufactured by adding *Lactobacillus acidophilus,* or may be naturally secreted or artificially produced from *Lactobacillus acidophilus,* but the present invention is not limited thereto.

In the present invention, the vesicles isolated by the above method may have an average diameter of 10 to 1000 nm, 10 to 900 nm, 10 to 800 nm, 10 to 700 nm, 10 to 600 nm, 10 to 500 nm, 10 to 400 nm, 10 to 300 nm, 10 to 220 nm, 10 to 200 nm, 10 to 100 nm, 10 to 90 nm, 10 to 80 nm, 10 to 70 nm, 10 to 60 nm, 10 to 50 nm, 10 to 40 nm, or 20 to 40 nm, but the present invention is not limited thereto.

The term "being included as an active ingredient" used herein refers to including a material in a sufficient amount to achieve the efficacy or activity of the *Lactobacillus acidophilus-derived* vesicles.

In the present invention, the inflammatory disease may comprise one or more inflammatory skin diseases selected from the group consisting of eczema, atopic dermatitis, psoriasis, acne, alopecia, contact dermatitis, seborrheic dermatitis, and mastocytosis;
one or more inflammatory oral diseases selected from the group consisting of caries, gingivitis, and periodontitis;
one or more inflammatory gastrointestinal diseases selected from the group consisting of ulcerative colitis, Crohn's disease, intestinal Behcet's disease, indeterminate colitis, chronic gastritis, chronic peptic ulcers, celiac disease, and inflammatory bowel disease;
one or more inflammatory liver-biliary-pancreatic diseases selected from the group consisting of non-alcoholic steatohepatitis (NASH), alcoholic steatohepatitis, liver cirrhosis, cholangitis, cholecystitis, and pancreatitis;
one or more inflammatory nasal diseases selected from the group consisting of rhinitis, paranasal sinusitis, and sinusitis;
one or more inflammatory lung diseases selected from the group consisting of bronchitis, asthma, chronic obstructive pulmonary disease (COPD), emphysema, cystic fibrosis, pneumonia, chronic interstitial pneumonitis, bronchopulmonary dysplasia, and idiopathic pulmonary fibrosis;
one or more inflammatory cardiovascular diseases selected from the group consisting of hyperinsulinemia, dyslipidemia, arrhythmia, atherosclerosis, angina, metabolic syndrome, myocardial infarction, cardiomyopathy, stroke, ischemic heart disease, heart failure, thrombosis, and atherosclerosis;
one or more inflammatory musculoskeletal diseases selected from the group consisting of atony, muscular atrophy, muscular dystrophy, myasthenia gravis, cachexia, gout, sarcopenia, osteoporosis, Paget's disease, rheumatoid arthritis, and osteoarthritis;
one or more inflammatory central nervous system diseases selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), anxiety disorder, post-traumatic stress disorder (PTSD), panic disorder, depression, autism spectrum disorders, attention deficit/hyperactivity disorder (ADHD), schizophrenia, dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy; and
one or more chronic inflammatory eye diseases selected from the group consisting of proliferative vitreoretinopathy, macular degeneration, pigmentary retinopathy, diabetic retinopathy, choroidal neovascularization, neovascular glaucoma, ischemic optic nerve disorders, retinopathy of prematurity, epidemic keratoconjunctivitis, neovascular iris disease, retrolental fibroplasia, atopic keratitis, superior corneal limbal keratitis, psoriasis keratitis, phlyctenular keratoconjunctivitis, scleritis, and diabetic macular edema, but the present invention is not limited thereto.

In the present invention, tumor necrosis factor α (TNF-α) is an intracellular mediator of immune responses, produced by various cells including activated macrophages and monocytes. Responses caused by TNF-α are initiated through interaction with two distinct TNF-α cell surface receptors, that is, TNFαR1 and TNFαR2. TNF-α binds to such cell surface receptors, causing the activation of transcription factors, such as nuclear factor κB (NPκB) regulating the expression of various immune and inflammatory response genes. Upon binding to TNF-α, a TNF-α receptor interacts with various intracellular signal translation proteins through a cytoplasmic domain. There are a variety of disease states associated with regulatory pathways controlled by TNF-α binding. In a specific example, TNF-α binding triggers an inflammatory response, and ultimately results in a disease state. Accordingly, the development of means for preventing diseases associated with TNF-α receptor binding is required. Particularly, there is a need to identify a method of preventing the activation of an inflammatory response that can be initiated by TNF-α activation. Meanwhile, when cytokines such as IL-23, IL-17, IL-6, and TNF-α secreted by differentiated T immune cells are excessively expressed due to hypersensitivity of the immune system or a patient's autoimmune abnormality due to a chronic inflammatory disease, an inflammatory response occurs.

The amount of the vesicles in the composition of the present invention may be appropriately adjusted depending on the symptoms of a disease, the degree of progression of symptoms, the condition of a patient, and the like, and may range from, for example, 0.0001 wt% to 99.9 wt% or 0.001 wt% to 50 wt% with respect to a total weight of the composition, but the present invention is not limited thereto. The amount ratio is a value based on the amount of dried product from which a solvent is removed.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the patient's age, sex, and body weight, and generally, 0.001 to 150 mg of the composition and preferably, 0.01 to 100 mg of the composition, per 1 kg of the body weight, may be administered daily or every other day or may be administered once to three times a day. However, since the effective amount may be increased or decreased depending on the administration route, the severity of obesity, gender, body weight, age, and the like, the dosage is not intended to limit the scope of the present invention in any way.

As used herein, the "subject" refers to a subject in need of treatment of a disease, and more specifically, refers to a mammal such as a human or a non-human primate, a mouse, a rat, a dog, a cat, a horse, and a cow, but the present invention is not limited thereto.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

In addition, the present invention is to provide a food composition for preventing or alleviating an inflammatory disease, comprising *Lactobacillus acidophilus-derived* vesicles as an active ingredient.

The food composition may be a health functional food composition, but is not limited thereto.

The vesicles according to the present invention may be used by adding the vesicles as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range, and the vesicles have no problem in terms of stability, so the active ingredient may be used in an amount more than the above-mentioned range.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not significantly important, but is generally selected within a range of 0.01 to 0.20 part by weight per 100 parts by weight of the composition of the present invention.

In addition, the present invention is to provide a cosmetic composition for preventing or alleviating an inflammatory skin disease, comprising *Lactobacillus acidophilus-derived* vesicles as an active ingredient.

A formulation for the cosmetic composition according to the present invention may include a skin lotion, a skin softener, a skin toner, an astringent, a lotion, a milk lotion, a moisturizing lotion, a nourishing lotion, a massage cream, a nourishing cream, a mist, a moisturizing cream, a hand cream, a hand lotion, a foundation, an essence, a nourishing essence, a pack, soap, a cleansing foam, a cleansing lotion, a cleansing cream, a cleansing oil, a cleansing balm, a body lotion or a body cleanser.

A cosmetic composition of the present invention may further include a composition selected from the group consisting of water-soluble vitamins, oil-soluble vitamins, polymer peptides, polymeric polysaccharides, and sphingolipids.

The water-soluble vitamin may be any substance that is blendable with cosmetics, but examples thereof include vitamin B1, vitamin B2, vitamin B6, pyridoxine, pyridoxine hydrochloride, vitamin B 12, pantothenic acid, nicotinic acid, nicotinic acid amide, folic acid, vitamin C, vitamin H, and the like, and salts thereof (thiamine hydrochloride, sodium ascorbate, and the like) or derivatives thereof (sodium ascorbic acid-2-phosphate, magnesium ascorbic acid-2-phosphate, and the like) are also included in water-soluble vitamins that may be used in the present invention. These water-soluble vitamins may be obtained by a conventional method such as microbial transformation, purification from a microbial culture, an enzyme method, or a chemical synthesis method.

The oil-soluble vitamins may be any substance that is blendable with cosmetics, but examples thereof include vitamin A, carotene, vitamin D2, vitamin D3, vitamin E (d1-α-tocopherol, d-α-tocopherol), or the like, and derivatives thereof (e.g., ascorbyl palmitate, ascorbyl stearate, ascorbyl dipalmitate, d1-α-tocopherol acetate, d1-α-tocopherol nicotinate, vitamin E, DL-pantothenyl alcohol, D-pantothenyl alcohol, pantothenyl ethylether) may also be included in the oil-soluble vitamins used in the present invention. These oil-soluble vitamins may be obtained by a conventional method such as microbial transformation, purification from a microbial culture, or enzymatic or chemical synthesis.

The polymer peptides may be any substance that is blendable with cosmetics, but examples thereof may include collagen, hydrolyzed collagen, gelatin, elastin, hydrolyzed elastin, and keratin. The polymer peptides may be purified and obtained by any conventional method such as purification from a microbial culture, an enzyme method, or a chemical synthesis method, or may generally be used by being purified from natural substances such as the dermis of a pig, a cow, or the like and silk fiber of silkworms.

The polymeric polysaccharides may be any substance that is blendable with cosmetics, and examples thereof may include hydroxyethyl cellulose, xanthan gum, sodium hyaluronate, and chondroitin sulfate or salts thereof (sodium salts). For example, chondroitin sulfate or salts thereof may generally be purified from mammals or fish and used.

The sphingolipids may be any substance that is blendable with cosmetics, and examples thereof may include ceramide, phytosphingosine, and sphingoglycolipid. The sphingolipids may be purified, by a conventional method, from mammals, fish, shellfish, yeast, or plants, or may be obtained by a chemical synthesis method.

The cosmetic composition of the present invention may include, as necessary, other ingredients mixed in conventional cosmetics along with the above essential ingredients.

Examples of additional ingredients to be mixed may include lipid components, a humectant, an emollient, a surfactant, organic and inorganic pigments, organic powder, a UV absorbent, a preservative, a sanitizer, an antioxidant, a plant extract, a pH adjuster, alcohol, pigments, flavors, a blood circulation promoter, a cooling agent, an anti-diaphoretic, and purified water.

The lipid components may include, for example, ester lipids, hydrocarbon lipids, silicone lipids, fluorine lipids, animal fats, vegetable oil, or the like.

The ester lipids may include, for example, glyceryl tri 2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linolate, isopropyl linolate, ethyl oleate, isocetyl myristate, isostearyl myristate, isostearyl palmitate, octyldodecyl myristate, isocetyl isostearate, diethyl sebacate, diisopropyl adipate, isoalkyl neopentanate, tri(capryl, capric acid)glyceryl, trimethylolpropane tri 2-ethylhexanoate, trimethylolpropane triisostearate, pentaerythritol tetra 2-ethylhexanoate, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linolate, isopropyl isostearate, cetostearyl 2-ethylhexanoate, stearyl 2-ethylhexanoate, hexyl isostearate, ethyleneglycol dioctanoate, ethyleneglycol dioleate, propyleneglycol dicaprinate, propyleneglycol di(caprylate, caprinate), propyleneglycol dicaprylate, neopentylglycol dicaprinate, neopentylglycol dioctanoate, glyceryl tricaprylate, glyceryl triundecylate, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerin ester oleate, polyglycerin ester isostearate, triisocetyl citrate, triisoalkyl citrate, triisooctyl citrate, lauryl lactate, myristyl lactate, cetyl lactate, octyldecyl lactate, triethyl citrate, acetyltriethyl citrate, acetyltributyl citrate, trioctyl citrate, diisostearyl malate, 2-ethylhexyl hydroxystearate, di 2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoyl hydroxystearate, stearoyl 12-stearoyl hydroxystearate, isostearyl 12-stearoyl hydroxystearate, and the like.

The hydrocarbon lipids may include, for example, squalene, liquid paraffin, alpha-olefin oligomers, isoparaffin, ceresine, paraffin, liquid isoparaffin, polybutene, microcrystalline wax, Vaseline, and the like.

The silicone lipids may include, for example, polymethyl silicon, methylphenyl silicon, methyl cyclopolysiloxane, octamethyl polysiloxane, decamethyl polysiloxane, dodecamethyl cyclosiloxane, dimethylsiloxane/methylcetyloxysiloxane copolymers, dimethylsiloxane/methylstearoxysiloxane copolymers, alkyl-modified silicon oil, amino-modified silicon oil, and the like.

The fluorine lipids may include perfluoropolyether and the like.

The animal or vegetable oil may include avocado oil, almond oil, olive oil, sesame oil, rice bran oil, safflower oil, soybean oil, corn oil, rape flower oil, apricot kernel oil, palm kernel oil, palm oil, castor oil, sunflower oil, grape seed oil, cotton seed oil, coconut oil, tallow nut oil, wheat germ oil, rice germ oil, Shea butter, evening primrose oil, macadamia nut oil, meadow foam seed oil, yolk oil, beef tallow, hemp seed oil, mink oil, orange roughy oil, jojoba oil, candelilla wax, carnauba wax, liquid lanolin, dehydrated castor oil, and the like.

The humectant may include water-soluble low molecular humectants, oil-soluble molecular humectants, water-soluble polymers, oil-soluble polymers, and the like.

The water-soluble low molecular humectants may include serine, glutamine, sorbitol, mannitol, pyrrolidone-sodium carboxylate, glycerin, propylene glycol, 1,3-butylene glycol, ethylene glycol, polyethylene glycol B (degree of polymerization: n=2 or higher), polypropylene glycol (degree of polymerization: n=2 or higher), polyglycerin B (degree of polymerization: n=2 or higher), lactic acid, lactates, and the like.

The oil-soluble low molecular humectants may include cholesterol, cholesterol ester, and the like.

The water-soluble polymers may include carboxyvinyl polymers, polyasparaginic acid salts, tragacanth, xanthan gum, methyl cellulose, hydroxymethyl cellulose, hydroxylethyl cellulose, hydroxypropyl cellulose, carboxymethyl cellulose, water-soluble chitin, chitosan, dextrin, and the like.

The oil-soluble polymers may include, for example, polyvinyl pyrrolidone/eicosen copolymers, polyvinyl pyrrolidone/hexadecene copolymers, nitrocellulose, dextrin fatty acid ester, silicone polymers, and the like.

The emollients may include, for example, long chain cholesterylester acyl glutamate, cholesteryl hydroxystearate, 12-hydroxystearic acid, stearic acid, rosin acid, lanolin fatty acid cholesteryl ester, and the like.

The surfactants may include, for example, non-ionic surfactants, anionic surfactants, cationic surfactants, amphoteric surfactants, and the like.

The non-ionic surfactants may include self-emulsion type glycerin monostearate, propyleneglycol fatty acid ester, glycerin fatty acid ester, polyglycerin fatty acid ester, sorbitan fatty acid ester, polyoxyethylene (POE) sorbitan fatty acid ester, POE sorbit fatty acid ester, POE glycerin fatty acid ester, POE alkylethers, POE fatty acid ester, POE dehydrated castor oil, POE castor oil, polyoxyethylene/polyoxypropylene (POE/POP) copolymers, POE/POP alkylethers, polyether-modified silicone, alkanolamide laurate, alkylamine oxide, hydrated soy phospholipids, and the like.

The anionic surfactants may include fatty acid soap, α-acylsulfonate, alkyl sulfonates, alkylallyl sulfonates, alkylnaphthalene sulfonates, alkyl sulfates, POE alkylether sulfates, alkylamide sulfates, alkyl phosphates, POE alkyl phosphates, alkylamide phosphates, alkyloyl alkyltaurin salts, N-acylamino acid salts, POE alkylether carboxylates, alkyl sulfosuccinates, sodium alkyl sulfoacetates, acylated hydrolyzed collagen peptide salts, perfluoroalkyl ester phosphates, and the like.

The cationic surfactants may include, for example, alkyltrimethylammonium chloride, stearyltrimethylammonium chloride, steraryltrimethylammonium bromide, cetostearyl trimethylammonium chloride, distearyl dimethylammonium chloride, stearylaryl dimethylbenzylammonium chloride, behenyltrimethylammonium bromide, benzalkonium chloride, diethylaminoethylamide stearate, dimethylaminopropylamide stearate, quaternary ammonium salts of lanolin derivatives, and the like.

The amphoteric surfactants may include carboxybetaine, amidebetaine, sulfobetaine, hydroxysulfobetaine, amidesulfobetaine, phosphobetaine, aminocarboxylate, imidazoline derivatives, amideamine-based amphoteric surfactants, and the like.

The organic and inorganic pigments may include: inorganic pigments such as silicic acid, anhydrous silicic acid, magnesium silicate, talc, sericite, mica, kaolin, bengala, clay, bentonite, titanium dioxide-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine, chromium oxide, chromium hydroxide, calamine, and combinations thereof; organic pigments such as polyamide, polyester, polypropylene, polystyrene, polyurethane, vinyl resin, urea resin, phenol resin, fluorine resin, silicon resin, acryl resin, melamine resin, epoxy resin, polycarbonate resin, divinyl benzene/styrene copolymers, silk powder, cellulose, CI pigment yellow, and CI pigment orange; and composite pigments of inorganic and organic pigments.

The organic powder may include: metallic soap such as calcium stearate; metal salts of alkyl phosphoric acid such as zinc sodium cetylate, zinc laurylate, and calcium laurylate; polymetallic salts of acylamino acid such as calcium N-lauroyl-beta-alanine, zinc N-lauroyl-beta-alanine, and calcium N-lauroylglycine; polymetallic salts of amide sulfonates such as calcium N-lauroyl-taurine and calcium N-palmitoyl-taurine.; N-acyl alkaline amino acids such as N-epsilon-lauroyl-L-lysine, N-epsilon-palmitoyl lysine, N-α-palmitoylol nitin, N-α-lauroyl arginine, and N-α-dehydrated tallow fatty acid acyl arginine; N-acyl polypeptides such as N-lauroyl glycylglycine; α-amino fatty acids such as α-aminocaprylic acid and α-aminolauric acid; polyethylene; polypropylene; nylon; polymethylmethacrylate; polystyrene; divinylbenzene/styrene copolymers; ethylene tetrafluoride; and the like.

The UV absorbents may include para-aminobenzoic acid, ethyl para-aminobenzoate, amyl para-aminobenzoate, octyl para-aminobenzoate, ethyleneglycol salicylate, phenyl salicylate, octyl salcylate, benzyl salicylate, butylphenyl salicylate, homomentyl salicylate, benzyl cinnamate, para-methoxycinnamic acid-2-ethoxylethyl, octyl paramethoxycinnamate, mono-2-ethylhexaneglyceryl diparamethoxycinnamate, isopropyl paramethoxycinnamate, diisopropyl/diisopropyl cinnamic acid ester mixtures, urocanic acid, ethyl urocanate, hydroxymethoxybenzophenone, hydroxymethoxybenzophenone sulfonic acid and salts thereof, dihydroxymethoxybenzophenone, sodium dihydroxymethoxybenzophenone disulfonate, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, 2-(2-hydroxy-5-methylphenyl)benzotriazole, and the like.

The sanitizers may include hinokitiol, trichloric acid, trichlorohydroxydiphenylether, chlorohexidine gluconate, phenoxyethanol, resorcine, isopropylmethylphenol, azulene, salicylic acid, zinc pyrithione, benzalkonium chloride, light sensitive element No. 301, sodium mononitroguaiacol, undecylenic acid, and the like.

The antioxidants may include butylhydroxyanisole, propyl gallate, elisorbic acid, and the like.

The pH adjusters may include citric acid, sodium citrate, malic acid, sodium malate, fumaric acid, sodium fumarate, succinic acid, sodium succinate, sodium hydroxide, sodium monohydrophosphate, and the like.

The alcohols may include higher alcohols such as cetyl alcohol.

In addition, additional ingredients to be mixed are not limited to the above examples, and any one of the above ingredients may be mixed within a range that does not adversely affect the objectives and effects of the present invention, but may range from 0.01 wt% to 5 wt% or 0.01 wt% to 3 wt% with respect to the total weight of the composition.

For lotion, paste, cream, or gel preparations of the present invention, as a carrier ingredient, animal fiber, vegetable fiber, wax, paraffin, starch, tragacanth, a cellulose derivative, polyethylene glycol, silicon, bentonite, silica, talc, zinc oxide, or the like may be used.

For powder or spray preparations of the present invention, lactose, talc, silica, aluminum hydroxide, calcium silicate, or polyamide powder may be used as a carrier ingredient. In particular, in the case of spray preparations, the composition may further include a propellant such as chlorofluorohydrocarbon, propane/butane, or dimethyl ether.

For solution or emulsion preparations of the present invention, a solvent, a solubilizing agent, or an emulsifying agent may be used as a carrier ingredient, and the carrier ingredient may be, for example, water, ethanol, isopropanol, ethyl carbonate, ethyl acetate, benzyl alcohol, benzyl benzoate, propylene glycol, 1,3-butyl glycol oil, a glycerol aliphatic ester, polyethylene glycol, or a sorbitan fatty acid ester.

For suspension preparations of the present invention, as a carrier ingredient, a liquid diluent such as water, ethanol, or propylene glycol, a suspending agent such as ethoxylated isostearyl alcohol, polyoxyethylene sorbitol ester, or polyoxyethylene sorbitan ester, micro-crystalline cellulose, aluminum methahydroxide, bentonite, agar, tragacanth, or the like may be used.

For surfactant-containing cleansing preparations of the present invention, as a carrier ingredient, an aliphatic alcohol sulfate, an aliphatic alcohol ether sulfate, a sulfosuccinate monoester, isethionate, imidazolinium derivatives, methyltaurate, sarcosinate, fatty acid amide ether sulfate, alkylamidobetaine, an aliphatic alcohol, a fatty acid glyceride, a fatty acid diethanol amide, vegetable oil, a lanolin derivative, an ethoxylated glycerol fatty acid ester, or the like may be used.

In addition, the present invention may be provided as an inhalant composition for preventing or treating an inflammatory disease or a cancer, comprising *Lactobacillus acidophilus-derived* vesicles as an active ingredient.

An inhalant may be formulated by a method known in the art, and may be conveniently applied in the form of a pressurized pack, a nebulizer, or an aerosol spray using a suitable propellant, for example, dichlorofluoromethane, trichlorofluoromethane, dichlorotetrafluoroethane, carbon dioxide, or other suitable gases. In the case of a pressurized aerosol, a dosage unit may be determined by providing a valve delivering a metered amount. For example, a gelatin capsule and cartridge used in an inhaler or nebulizer may be formulated to contain a powder mixture of a compound and a suitable powder base such as lactose or starch.

In addition, the present invention relates to a composition for delivering a disease-treating drug, which comprises *Lactobacillus acidophilus-derived* vesicles as an active ingredient.

The term "drug delivery" used herein refers to any means or act of loading and delivering a drug in the composition according to the present invention in order to deliver the drug to a specific organ, tissue, cell or organelle.

In the present invention, the composition for drug delivery may deliver a drug to one or more organs selected from the group consisting of the stomach, the small intestine, the large intestine, the lungs, the liver, and the brain, but the present invention is not limited thereto.

In the present invention, the disease may be one or more selected from the group consisting of a stomach disease, an intestinal disease, a lung disease, a liver disease, and a central nervous system disease, but the present invention is not limited thereto.

In addition, the present invention provides a method of delivering a drug for treating a disease, which comprises administering a composition comprising *Lactobacillus acidophilus-derived* vesicles as an active ingredient to a subject in need thereof.

In addition, the present invention provides a use of a composition comprising *Lactobacillus acidophilus-derived* vesicles carrying a drug for treating a disease as an active ingredient for delivering a disease-treating drug.

In addition, the present invention provides a drug delivery system, which comprises *Lactobacillus acidophilus-derived* vesicles.

In addition, the present invention provides a use of *Lactobacillus acidophilus-*derived vesicles for preparing an agent for drug delivery in cells.

### [Modes of the Invention]

Hereinafter, preferred Examples for helping the understanding of the present invention will be suggested. However, the following Examples are provided only to more easily understand the present invention, and the contents of the present invention are not limited by the following Examples.

### [Examples]

### Example 1: Isolation of Lactobacillus acidophilus-derived vesicles

To isolate *Lactobacillus acidophilus-derived* vesicles, *Lactobacillus acidophilus* was inoculated in a deMan-Rogosa and Sharpe (MRS) medium and cultured at 37 °C and 200 rpm until an absorbance (OD₆₀₀ₙₘ) became 1.0 to 1.5. Subsequently, *Lactobacillus acidophilus* was reinoculated in a Luria Bertani (LB) medium and cultured. In addition, the culture medium containing the bacterial cells was recovered and centrifuged at 10,000 g and 4 °C for 20 minutes to obtain a supernatant from which the bacterial cells were removed. The obtained supernatant was then filtrated using a 0.22 µm filter. The filtered supernatant was concentrated to a volume of 50 mL or less using a 100 kDa Pellicon 2 Cassette filter membrane (Merck Millipore) and a MasterFlex pump system (Cole-Parmer). Afterward, the concentrated supernatant was filtrated again using a 0.22 µm filter, and *Lactobacillus acidophilus-derived* vesicles were isolated. To quantify the vesicles contained in the supernatant, the amount of protein contained in the vesicles was measured using the Pierce BCA Protein Assay kit (Thermo Fisher Scientific).

### Example 2: Pharmacokinetic properties of Lactobacillus acidophilus-derived vesicles

To confirm the pharmacokinetic properties of absorption, distribution, and excretion patterns after orally administering the *Lactobacillus acidophilus-derived* vesicles, an experiment was conducted in the following manner. *Lactobacillus acidophilus-derived* vesicles labeled with the fluorophore Cy7 were orally administered to a mouse, and after 0, 1, 3, 6, 24, and 48 hours, entire mouse images and distribution patterns to organs obtained after extracting various organs were evaluated. To observe fluorescent signals, optical imaging equipment (Davinch-In vivo Fluoro Chemi (western); Davinch-K) was used.

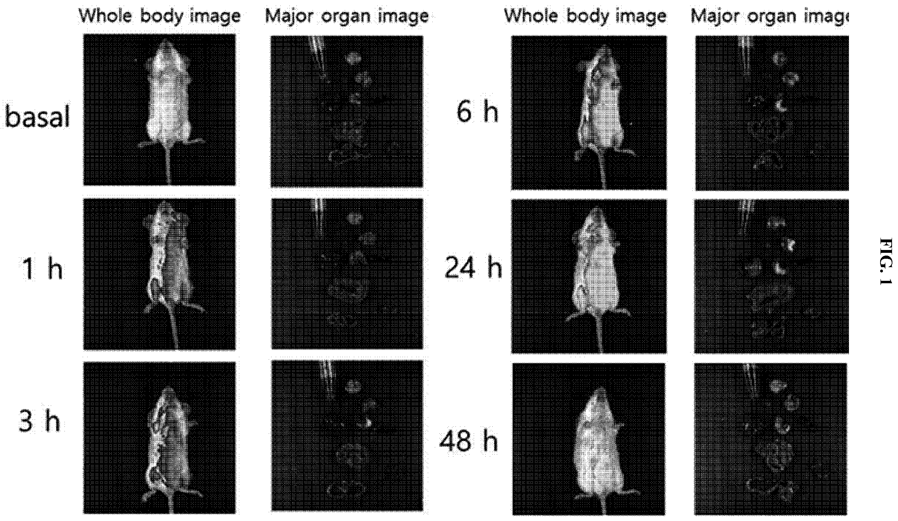

As shown in FIG. 1, it was confirmed that the vesicles were absorbed into the body 1 hour after vesicle administration and continued until 24 hours after administration, and most of the vesicles were excreted after 48 hours. In addition, when the distribution patterns to major organs were evaluated after orally administering the vesicles, it was seen that the vesicles were distributed to various organs 1 hour after oral administration.

After orally administering the vesicles, the absorption, distribution, and excretion patterns in the stomach, small intestine, and large intestine were evaluated over time.

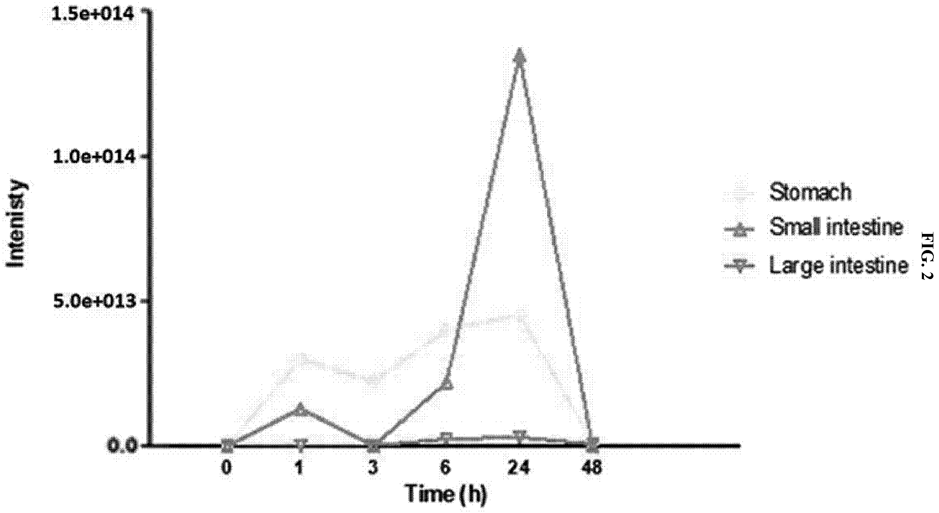

As shown in FIG. 2, at the first hour after administration, the vesicles were mainly absorbed into the stomach and small intestine, and distributed in tissue, and after 48 hours, most of the vesicles were excreted.

In addition, after orally administering the vesicles, the absorption, distribution, and excretion patterns in the lungs and liver were evaluated over time.

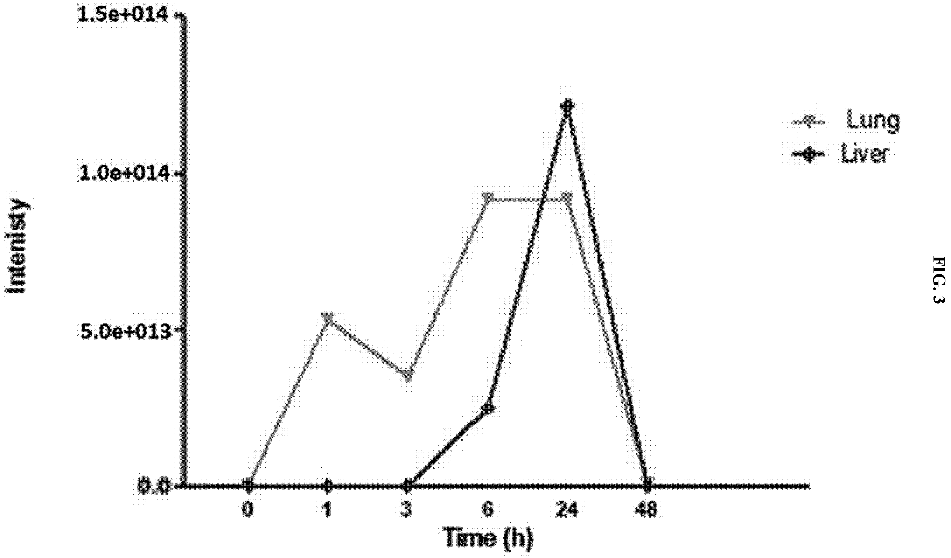

As shown in FIG. 3, at the first hour after administration, the vesicles were absorbed into the lungs and distributed into lung tissue, and mostly excreted 48 hours after administration. On the other hand, absorption into the liver occurred at 3 hours after vesicle administration, and mostly excreted 48 hours after administration.

In addition, after orally administering the vesicles, absorption, distribution, and excretion patterns in the central nervous system were evaluated over time.

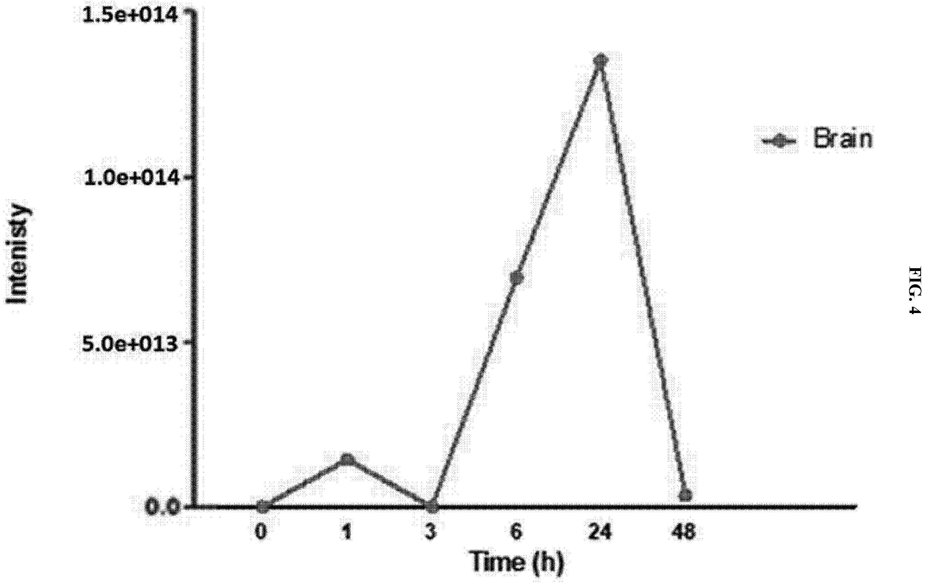

As shown in FIG. 4, at 3 hours after administration, the vesicles were mainly absorbed into brain tissue, mostly distributed in the brain tissue at 24 hours after administration, and mostly excreted 48 hours after administration.

In addition, after orally administering the vesicles, absorption, distribution and excretion patterns in the heart and kidneys were evaluated over time.

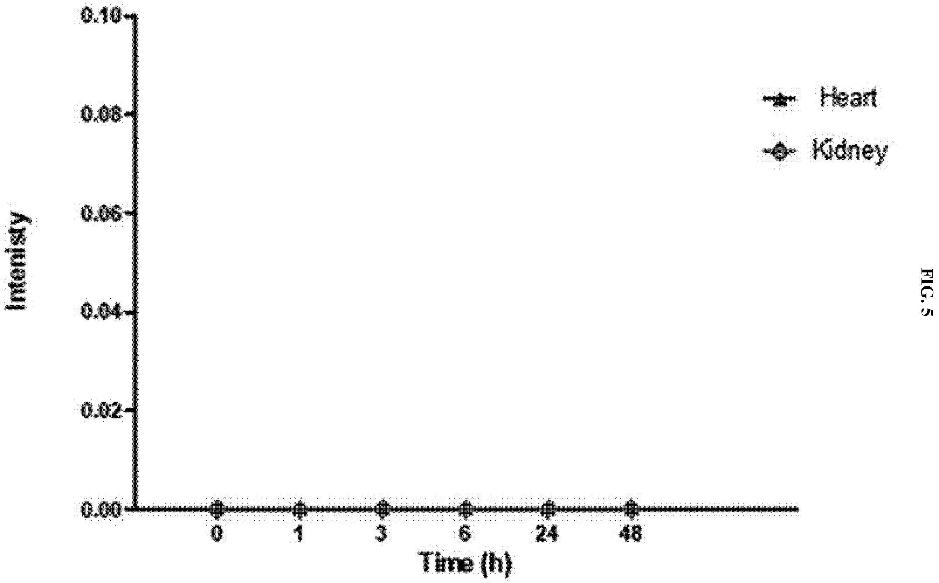

As shown in FIG. 5, the vesicles were not absorbed and distributed into heart and kidney tissue.

From the above result, it can be seen that when *Lactobacillus acidophilus-*derived vesicles were orally administered, the vesicles were mainly absorbed via the stomach and small intestine, moved to blood vessels through lymphatic vessels in gastrointestinal tissue, distributed into lung tissue via the pulmonary artery, and then distributed to the central nervous system and the liver via the systemic artery.

### Example 3: Comparison of inflammation-inducing effects of E. coli-derived vesicles and Lactobacillus acidophilus-derived vesicles

To evaluate the extent to which *Lactobacillus acidophilus-derived* vesicles induce inflammation by inflammatory cells, a mouse macrophage cell line Raw 264.7 was treated with the *Lactobacillus acidophilus-derived* vesicles at concentrations of 0.1, 1, and 10 µg/mL and allowed to react, and then the secretion amounts of TNF-α and IL-6, which are representative inflammatory mediators, causing inflammatory diseases, were measured. To evaluate the degree of inflammatory response, as a positive control pathogenic factor, *E. coli*-derived vesicles (*E. coli* EV) were treated for comparison. More specifically, after seeding 1 × 10⁵ of Raw 264.7 cells in a 48-well plate, *Lactobacillus acidophilus-derived* vesicles or E. coli-derived vesicles, diluted in serum-free DMEM were treated and cultured for 12 hours. Subsequently, the cell culture was put into a 1.5 mL tube and centrifuged at 3,000 g for 5 minutes to obtain only a supernatant, the supernatant was stored at -80 °C, and then the amounts of TNF-α and IL-6 were measured by ELISA (R&D Systems). For ELISA, capture antibodies (Abcam) were diluted in phosphate buffered saline (PBS), dispensed at 50 µL in each well of a 96-well plate, and then reacted at 4 °C for 16 hours. In addition, after washing three times with 100 µL of 0.05% Tween-20-containing PBS (PBST), 1% bovine serum albumin (BSA)-containing PBS (RD) was added at 100 µL per well to perform blocking at room temperature for 1 hour. In addition, 50 µL each of the sample to be tested and the standard were added at the predetermined concentration, reacted at room temperature for 2 hours, and then washed three times with PBST. In addition, detection antibodies were diluted in RD and added at 50 µL per well and allowed to react at room temperature for 2 hours, and after completely removing unbound antibodies by washing three times with PBST, 50 µL of streptavidin-HRP (R&D Systems) diluted 1/40 in RD was added to each well and allowed to react at room temperature for 20 minutes. Finally, after washing three times with 100 µL of PBST, 50 µL of a TMB substrate (SurModics) was added per well, and when color development progressed after 5 to 20 minutes, 50 µL of a 1M sulfuric acid solution was added per well to stop the reaction. Absorbance was then measured at 450 nm using a SpectraMax M3 microplate reader (Molecular Devices).

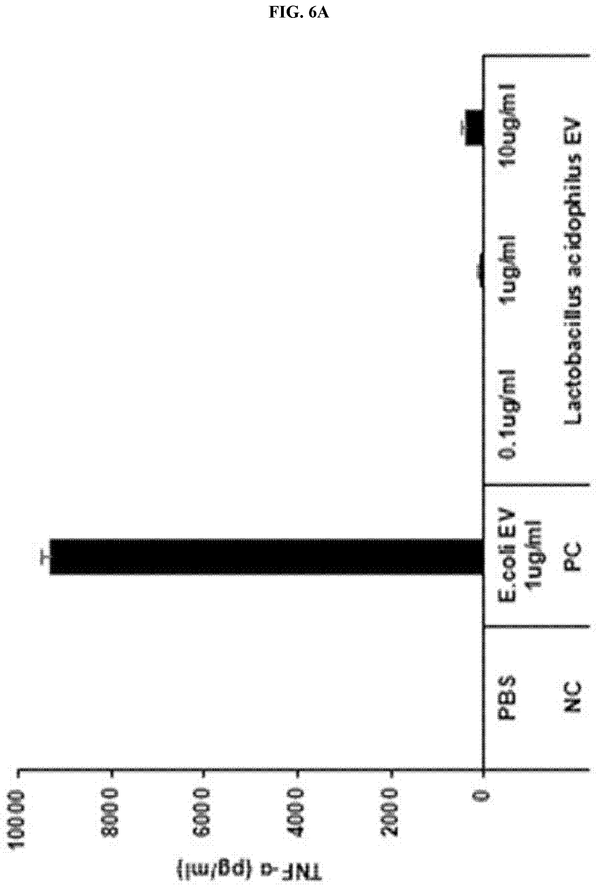

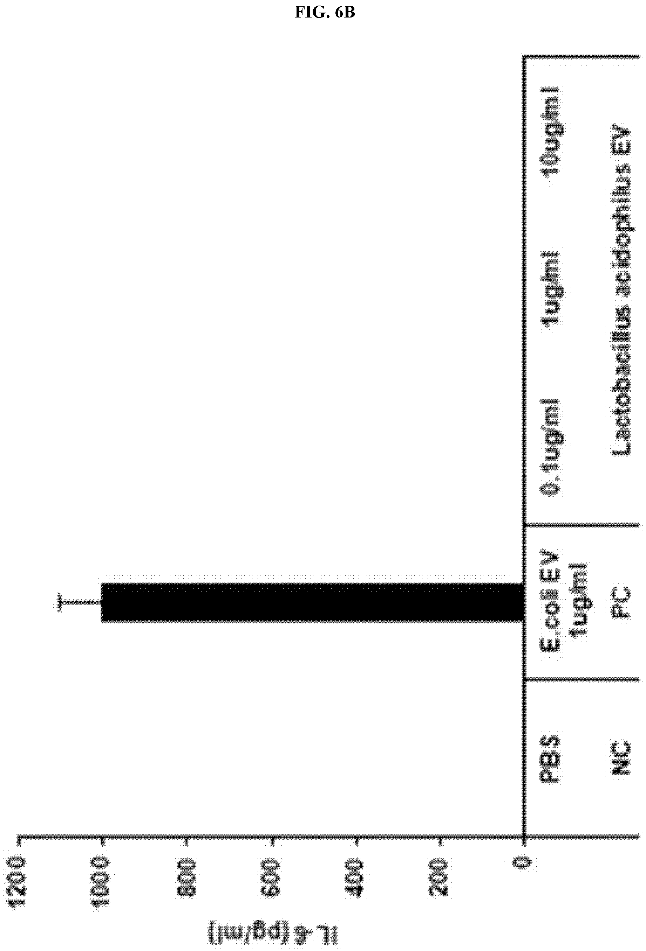

As a result, as shown in FIGS. 6A and 6B, when treated with 1 µg/mL of *E. coli*-derived vesicles, the secretion of TNF-α and IL-6 was induced in inflammatory cells, whereas when treated with the same amount of *Lactobacillus acidophilus-*derived vesicles, the secretion of TNF-α and IL-6 was not induced. In addition, even when inflammatory cells were treated with *Lactobacillus acidophilus-derived* vesicles at a concentration 10 times that of *E. coli*-derived vesicles, the secretion of TNF-α and IL-6 was hardly induced.

The above results show that *Lactobacillus acidophilus-derived* vesicles are significantly less effective in causing inflammation compared to *E. coli*-derived vesicles, which is a pathogenic factor.

### Example 4: Anti-inflammatory effect of Lactobacillus acidophilus-derived vesicles on pathogenic factor-induced inflammatory response

It is known that vesicles secreted by pathogenic bacteria are important pathogenic factors causing various inflammatory diseases. Accordingly, the therapeutic effect of *Lactobacillus acidophilus-derived* vesicles on the pathogenesis of an inflammatory disease caused by a pathogenic factor was confirmed. The mouse macrophage cell line Raw 264.7 was pretreated with *Lactobacillus acidophilus-*derived vesicles at concentrations of 0.1, 1, and 10 µg/mL and cultured, and then the inflammatory cells were treated with 1 µg/mL of *E. coli*-derived vesicles, followed by measuring the secretion amounts of TNF-α and IL-6. More specifically, the Raw 264.7 cells were seeded in a 48-well plate, treated with the *Lactobacillus acidophilus-*derived vesicles diluted with serum-free DMEM and cultured for 12 hours. Then, the cells were again treated with 1 µg/mL of *E*. *coli*-derived vesicles and cultured for additional 12 hours. The secretion amounts of TNF-α and IL-6 were measured in the same manner as in Example 3.

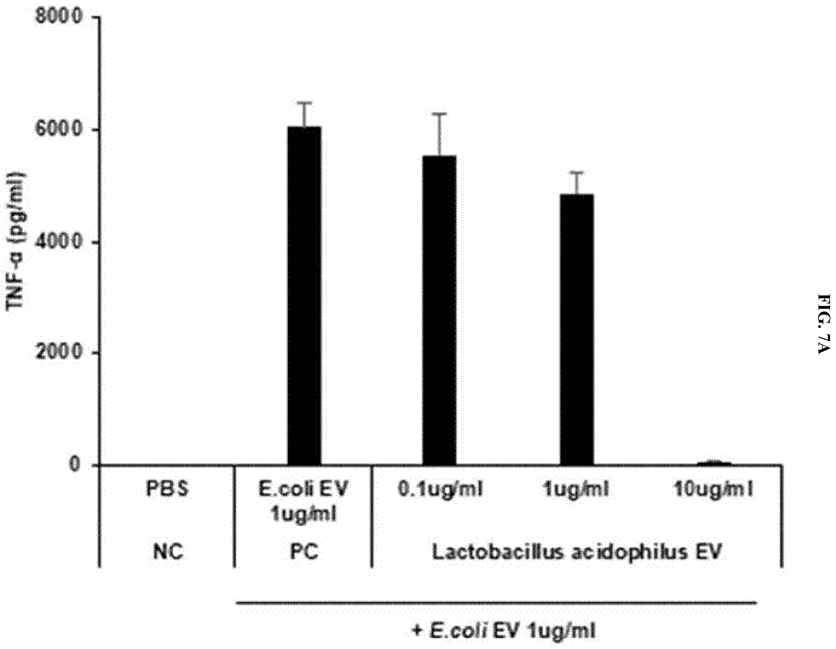

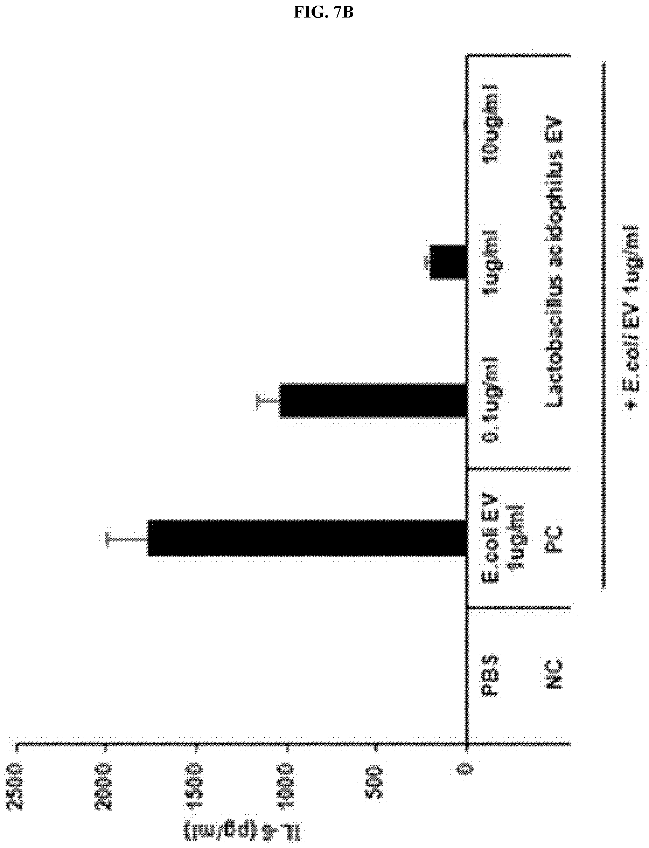

As a result, as shown in FIGS. 7A and 7B, in samples pretreated with *Lactobacillus acidophilus-derived* vesicles, it was confirmed that the secretion of TNF-α and IL-6 by E. coli-derived vesicles was inhibited in a dose-dependent manner.

The above result showed that *Lactobacillus acidophilus-derived* vesicles effectively inhibit inflammation induced by a pathogenic factor causing an inflammatory disease.

From the results, it was confirmed that the *Lactobacillus acidophilus-derived* vesicles of the present invention are absorbed and distributed into major organs and thus effectively inhibit the occurrence of a TNF-α or IL-6-mediated inflammatory disease, and the *Lactobacillus acidophilus-derived* vesicles of the present invention are expected to be used to alleviate, prevent, or treat an inflammatory disease.

The above-described description of the present invention is merely provided to exemplify the present invention, and it will be understood by those of ordinary skill in the art to which the present invention belongs that the present invention can be implemented in modified forms without departing from the essential features of the present invention. Therefore, the exemplary embodiments described above should be interpreted as illustrative and not limited in any aspect.

### [Industrial Applicability]

*Lactobacillus acidophilus-derived* vesicles according to the present invention can effectively inhibit the expression of TNF-α or IL-6, which is a mediator causing an inflammatory disease by a pathogenic factor, so they are expected to be widely used as an agent for alleviating, preventing, or treating various inflammatory diseases.

## Claims

1. A pharmaceutical composition for preventing or treating an inflammatory disease, comprising *Lactobacillus acidophilus-derived* vesicles as an active ingredient.

2. The pharmaceutical composition of claim 1, wherein the inflammatory disease is selected from the group consisting of the following diseases:
one or more inflammatory skin diseases selected from the group consisting of eczema, atopic dermatitis, psoriasis, acne, alopecia, contact dermatitis, seborrheic dermatitis, and mastocytosis;
one or more inflammatory oral diseases selected from the group consisting of caries, gingivitis, and periodontitis;
one or more inflammatory gastrointestinal diseases selected from the group consisting of ulcerative colitis, Crohn's disease, intestinal Behcet's disease, indeterminate colitis, chronic gastritis, chronic peptic ulcers, celiac disease, and inflammatory bowel disease;
one or more inflammatory liver-biliary-pancreatic diseases selected from the group consisting of non-alcoholic steatohepatitis (NASH), alcoholic steatohepatitis, liver cirrhosis, cholangitis, cholecystitis, and pancreatitis;
one or more inflammatory nasal diseases selected from the group consisting of rhinitis, paranasal sinusitis, and sinusitis;
one or more inflammatory lung diseases selected from the group consisting of bronchitis, asthma, chronic obstructive pulmonary disease (COPD), emphysema, cystic fibrosis, pneumonia, chronic interstitial pneumonitis, bronchopulmonary dysplasia, and idiopathic pulmonary fibrosis;
one or more inflammatory cardiovascular diseases selected from the group consisting of hyperinsulinemia, dyslipidemia, arrhythmia, atherosclerosis, angina, metabolic syndrome, myocardial infarction, cardiomyopathy, stroke, ischemic heart disease, heart failure, thrombosis, and atherosclerosis;
one or more inflammatory musculoskeletal diseases selected from the group consisting of atony, muscular atrophy, muscular dystrophy, myasthenia gravis, cachexia, gout, sarcopenia, osteoporosis, Paget's disease, rheumatoid arthritis, and osteoarthritis;
one or more inflammatory central nervous system diseases selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), anxiety disorder, post-traumatic stress disorder (PTSD), panic disorder, depression, Autism spectrum disorders, attention deficit/hyperactivity disorder (ADHD), schizophrenia, dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy; and
one or more chronic inflammatory eye diseases selected from the group consisting of proliferative vitreoretinopathy, macular degeneration, pigmentary retinopathy, diabetic retinopathy, choroidal neovascularization, neovascular glaucoma, ischemic optic nerve disorders, retinopathy of prematurity, epidemic keratoconjunctivitis, neovascular iris disease, retrolental fibroplasia, atopic keratitis, superior corneal limbal keratitis, psoriasis keratitis, phlyctenular keratoconjunctivitis, scleritis, and diabetic macular edema.

3. The pharmaceutical composition of claim 1, wherein the inflammatory disease is mediated by TNF-α or IL-6.

4. The pharmaceutical composition of claim 1, wherein the vesicles have an average diameter of 10 to 300 nm.

5. The pharmaceutical composition of claim 1, wherein the vesicles are naturally secreted or artificially produced from *Lactobacillus acidophilus.*

6. The pharmaceutical composition of claim 1, wherein the vesicles are isolated from the culture medium of *Lactobacillus acidophilus* or food cultured by adding *Lactobacillus acidophilus.*

7. A food composition for preventing or alleviating an inflammatory disease, comprising *Lactobacillus acidophilus-derived* vesicles as an active ingredient.

8. The food composition of claim 7, wherein the inflammatory disease is selected from the group consisting of the following diseases:
one or more inflammatory skin diseases selected from the group consisting of eczema, atopic dermatitis, psoriasis, acne, alopecia, contact dermatitis, seborrheic dermatitis, and mastocytosis;
one or more inflammatory oral diseases selected from the group consisting of caries, gingivitis, and periodontitis;
one or more inflammatory gastrointestinal diseases selected from the group consisting of ulcerative colitis, Crohn's disease, intestinal Behcet's disease, indeterminate colitis, chronic gastritis, chronic peptic ulcers, celiac disease, and inflammatory bowel disease;
one or more inflammatory liver-biliary-pancreatic diseases selected from the group consisting of non-alcoholic steatohepatitis (NASH), alcoholic steatohepatitis, liver cirrhosis, cholangitis, cholecystitis, and pancreatitis;
one or more inflammatory nasal diseases selected from the group consisting of rhinitis, paranasal sinusitis, and sinusitis;
one or more inflammatory lung diseases selected from the group consisting of bronchitis, asthma, chronic obstructive pulmonary disease (COPD), emphysema, cystic fibrosis, pneumonia, chronic interstitial pneumonitis, bronchopulmonary dysplasia, and idiopathic pulmonary fibrosis;
one or more inflammatory cardiovascular diseases selected from the group consisting of hyperinsulinemia, dyslipidemia, arrhythmia, atherosclerosis, angina, metabolic syndrome, myocardial infarction, cardiomyopathy, stroke, ischemic heart disease, heart failure, thrombosis, and atherosclerosis;
one or more inflammatory musculoskeletal diseases selected from the group consisting of atony, muscular atrophy, muscular dystrophy, myasthenia gravis, cachexia, gout, sarcopenia, osteoporosis, Paget's disease, rheumatoid arthritis, and osteoarthritis;
one or more inflammatory central nervous system diseases selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), anxiety disorder, post-traumatic stress disorder (PTSD), panic disorder, depression, Autism spectrum disorders, attention deficit/hyperactivity disorder (ADHD), schizophrenia, dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy; and
one or more chronic inflammatory eye diseases selected from the group consisting of proliferative vitreoretinopathy, macular degeneration, pigmentary retinopathy, diabetic retinopathy, choroidal neovascularization, neovascular glaucoma, ischemic optic nerve disorders, retinopathy of prematurity, epidemic keratoconjunctivitis, neovascular iris disease, retrolental fibroplasia, atopic keratitis, superior corneal limbal keratitis, psoriasis keratitis, phlyctenular keratoconjunctivitis, scleritis, and diabetic macular edema.

9. The food composition of claim 7, wherein the inflammatory disease is mediated by TNF-α or IL-6.

10. The food composition of claim 7, wherein the vesicles are naturally secreted or artificially produced from *Lactobacillus acidophilus.*

11. An inhalant composition for preventing or treating an inflammatory disease or a cancer, comprising *Lactobacillus acidophilus-derived* vesicles as an active ingredient.

12. A cosmetic composition for preventing or alleviating an inflammatory skin disease, comprising *Lactobacillus acidophilus-derived* vesicles as an active ingredient.

13. The cosmetic composition of claim 12, wherein the inflammatory skin disease is one or more selected from the group consisting of eczema, atopic dermatitis, psoriasis, acne, alopecia, contact dermatitis, seborrheic dermatitis, and mastocytosis.

14. The cosmetic composition of claim 12, wherein the inflammatory skin disease is mediated by TNF-α or IL-6.

15. A composition for delivering a disease-treating drug, which comprises *Lactobacillus acidophilus-derived* vesicles as an active ingredient.

16. The composition for delivering a disease-treating drug of claim 15, wherein the disease is one or more selected from the group consisting of a stomach disease, an intestinal disease, a lung disease, a liver disease, and a central nervous system disease.

17. The composition for delivering a disease-treating drug of claim 16, the central nervous system disease is one or more selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS), myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), anxiety disorder, post-traumatic stress disorder (PTSD), panic disorder, depression, Autism spectrum disorders, attention deficit/hyperactivity disorder (ADHD), schizophrenia, dementia with Lewy bodies (DLB), multi-infarct dementia (MID), frontotemporal lobar degeneration (FTLD), Pick's disease, corticobasal degeneration (CBD), progressive supranuclear palsy (PSP), age-related macular degeneration (AMD), dysosmia, deafness, and diabetic retinopathy.

18. A method for preventing or treating an inflammatory disease, the method comprising administering a composition comprising *Lactobacillus acidophilus-*derived vesicles as an active ingredient to a subject in need thereof.

19. A use of a composition comprising *Lactobacillus acidophilus-derived* vesicles as an active ingredient for preventing or treating an inflammatory disease.

20. A use of *Lactobacillus acidophilus-derived* vesicles for producing a drug for treating an inflammatory disease.

21. A method for preventing, alleviating or treating an inflammatory skin disease, the method comprising administering a composition comprising *Lactobacillus acidophilus-derived* vesicles as an active ingredient to a subject in need thereof.

22. A use of a composition comprising *Lactobacillus acidophilus-derived* vesicles as an active ingredient for preventing, alleviating or treating an inflammatory skin disease.

23. A use of *Lactobacillus acidophilus-derived* vesicles for preparing a drug for treating an inflammatory skin disease.

24. A method of delivering a drug for treating a disease, which comprises administering a composition comprising *Lactobacillus acidophilus-derived* vesicles as an active ingredient to a subject in need thereof.

25. A use of a composition comprising *Lactobacillus acidophilus-derived* vesicles as an active ingredient for delivering a disease-treating drug.

26. A drug delivery system, which comprises *Lactobacillus acidophilus-derived* vesicles.

27. A use of *Lactobacillus acidophilus-derived* vesicles for preparing an agent for drug delivery in cells.
